# EUROPÄISCHE PATENTSCHRIFT

(11) **EP 0 645 127 B1**
(45) Veröffentlichungstag und Bekanntmachung des Hinweises auf die Patenterteilung: **17.01.1996**
(21) Anmeldenummer: 94114466.9
(22) Anmeldetag: 14.09.1994
(51) Int. Cl.: A61F 2/46

(54) **Ausschlagwerkzeug für Gelenkprothesen**
Hip prostheses extractor with sliding mass
Outil à choc pour extraction d'une prothèse de hanche

(30) Priorität: 27.09.1993 DE 4332872
(43) Veröffentlichungstag der Anmeldung: 29.03.1995
(73) Patentinhaber: Szabo, Zsolt, D-80933 München (DE)
(72) Erfinder: Szabo, Zsolt, D-80993 München (DE); Seeberger, Georg, D-82396 Pähl (DE)
(74) Vertreter: Müller-Boré & Partner Patentanwälte

(56) Entgegenhaltungen:
- FR-A- 2 627 983
- FR-A- 2 686 016
- US-A- 4 222 382

## Beschreibung

Die vorliegende Erfindung betrifft ein Ausschlagwerkzeug für Gelenkprothesen, gemäß dem Oberbegriff des Anspruchs 1.

Ein derartiges Ausschlagwerkzeug ist beispielsweise aus der DE 42 20 970 A1 bekannt. Der Werkzeugkopf ist gekrümmt, um eine Abwinkelung des Prothesenschaftes gegenüber einer Prothesenmittelachse auszugleichen, so daß die im Knochen eingesetzte Gelenkprothese in Verlängerung der Prothesenmittelachse herausgeschlagen werden kann. Der Krümmungswinkel des Werkzeugkopfes entspricht daher im wesentlichen der Abwinkelung des Prothesenschaftes gegenüber der Prothesenmittelachse. Eine Fehlausrichtung zwischen Ausschlagkraftvektor und Prothesenmittelachse führt zu einer Splitterung des die Gelenkprothese umgebenden Knochens, was unerwünscht ist.

Das aus der DE 4220970 A1 bekannte Ausschlagwerkzeug hat eine Zunge, die auf einem außerhalb des Werkzeugkopfes gelagerten Hebelarm gebildet ist und zum Eingriff in eine im Prothesenschaft gebildete Ausnehmung bestimmt ist, um die Prothese am Werkzeugkopf zu verriegeln. Der Platzbedarf dieses Werkzeugs ist daher groß.

Die Prothesenschafte gängiger Gelenkprothesen sind nicht genormt und zeigen unterschiedliche Durchmesser. Das vorbekannte Ausschlagwerkzeug berücksichtigt derartige unterschiedliche Prothesenschaftdurchmesser jedoch nicht ausreichend und die Anpassung an verschiedene Durchmesser ist aufwendig. Bei Verwendung derartiger Ausschlagwerkzeuge bei Prothesenschaften geringeren Durchmessers wird der Prothesenschaft nicht immer spielfrei und formschlüssig im Werkzeugkopf verriegelt.

Ferner ist zu beachten, daß der im vorbekannten Ausschlagwerkzeug außerhalb des Werkzeugkopfes gelagerte Schwenkhebel den Platzbedarf des Werkzeugs erheblich erhöht. Am Operationsort steht jedoch nur ein begrenzter Raum für das Ausschlagwerkzeug zur Verfügung.

Aufgabe der vorliegenden Erfindung ist es daher, ein gemäß Oberbegriff des Anspruchs 1 ausgebildetes Ausschlagwerkzeug derart weiterzubilden, daß unterschiedliche Prothesendurchmesser im Werkzeugkopf leicht einstellbar sind. Eine weitere Aufgabe besteht darin, daß das erfindungsgemäße Ausschlagwerkzeug einen möglichst geringen Platz einnimmt, der zweckmäßigerweise auf den Werkzeugkopf beschränkt sein sollte.

Die Aufgabe wird gelöst durch ein Ausschlagwerkzeug nach Anspruch 1.

Erfindungsgemäß ist somit die Freigabeposition und die Eingriffsposition des in den Durchgang eingreifenden Sperrglieds der Riegeleinrichtung so einstellbar, daß Prothesenschafte unterschiedlichen Durchmessers in den Durchgang eingeführt und in Anlage mit dem Sperrglied gebracht werden können. Die in der Freigabeposition zwischen Sperrglied und den den Durchgang definierenden Teilen des Werkzeugskopfs definierte Querschnittsfläche läßt sich somit vergrößern und verkleinern. Die Freigabeposition des Sperrglieds ist durch spielfreie Anlage des Sperrglieds am Prothesenschaft definiert, wobei das Sperrglied von dieser Freigabeposition durch ein geeignetes Mittel in die Sperrposition gespannt und fest verriegelt wird. Die für die Betätigung des Sperrglieds zur Verstellung der Freigabeposition aufgebrachte Stellkraft wird durch Stellglieder im Werkzeugkopf übertragen, so daß eine außerhalb des Werkzeugkopfs angebrachte Stelleinrichtung entfällt und die Kraftübertragung im Werkzeugkopf selbst geschieht. Auf diese Weise ist der Platzbedarf des Werkzeugkopfs minimiert.

Es ist bevorzugt, daß der Werkzeugkopf stetig gekrümmt ist. Die stetige Krümmung des Werkzeugkopfs begünstigt die Wirkverbindung der Stellglieder, da die Stellkraft im wesentlichen axial übertragen werden kann. Die bei der Kraftübertragung wirkenden Normalkräfte sind bei einer stetigen Krümmung minimiert.

Der Werkzeugkopf hat bevorzugt eine im Durchgang abschließende Längsausnehmung, in der die Stellglieder und das Sperrglied angeordnet und geführt sind. Dadurch läßt sich der Werkzeugkopf bequem montieren und demontieren. Die den Werkzeugkopf bildenden Einzelteile lassen sich nach der Demontage leicht sterilisieren, was für medizinische Anwendungen große Bedeutung hat.

Die Stellglieder und das Sperrglied sind bevorzugt durch im Werkzeugkopf gebildete gegenüberliegende Nuten geführt. In diese Nuten greifen das Sperrglied und die Stellglieder mit entsprechenden Führungsnasen ein, so daß das Sperrglied und die Stellglieder axial in die Längsausnehmung des gabelförmigen Werkzeugkopfs eingeschoben und herausgezogen werden können.

Ein Stellglied ist bevorzugt mit einem Hebel verbunden, der zum Vorschub des Sperrglieds in die Verriegelungsposition zur lösbaren Blockierung des Sperrglieds in der Verriegelungsposition dient. Hierbei wird das Sperrglied in doppelter Funktion einmal zur Übertragung der Stellkraft und zum anderen zur Übertragung der Verriegelungskraft eingesetzt. Das Sperrglied wird aus der in Anlage mit dem Prothesenschaft definierten Freigabeposition durch axiale Verspannung gegen den Prothesenschaft gedrückt und in dieser Verriegelungsposition so verriegelt, daß das Sperrglied arretiert ist und nicht zurückweichen kann. Durch die Ausbildung des Stellglieds mit Hebel entfällt eine zusätzliche Verriegelungseinrichtung für das Sperrglied und eine platzsparende Minimalanordnung wird geschaffen.

Die Schwenkachse des Hebels ist bevorzugt im Stellglied gebildet, um eine zusätzliche Schwenkachse zu vermeiden.

Bevorzugt weist das Stellglied eine exzentrische Auflauffläche auf, die eine vom Schwenkwinkel abhängige axiale Vorschubposition des Sperrglieds definiert. Die Auflauffläche definiert die Anlage des mit Hebel ausgebildeten Stellglieds mit dem nachfolgenden Stellglied bzw. dem Sperrglied, so daß der unterschiedliche Abstand verschiedener winkelmäßig versetzter Punkte der Auflauffläche von der Schwenkachse einen unterschiedlichen Vorschub der Auflauffläche bei der Verschwenkung des Hebels zur Folge hat.

Das Stellglied weist bevorzugt zwei Führungsnasen auf, die längsverschieblich in den im Werkzeugkopf gebildeten Innennuten geführt sind, wobei die Führungsnasen die Schwenkachse des Hebels bilden. Die Führungsnasen haben einmal die Funktion, das Stellglied zur Übertragung der Stellkraft in den Nuten des Werkzeugkopfs zu führen. Zum anderen dienen sie als Schwenkachse des mit dem. Stellglied verbundenen Hebels, so daß wiederum eine platzsparende Minimalanordnung geschaffen ist.

Die Stellglieder sind in einer weiteren Ausführungsform durch Kugeln oder Rollen gebildet, die miteinander in Wirkverbindung stehen. Die Kugeln folgen der Krümmung des Werkzeugkopfs in besonders platz- und kraftsparender Weise, da sie im Vergleich zur Länge des Werkzeugkopfs geringen Durchmesser haben.

Bevorzugt ist mit dem Werkzeugkopf eine Betätigungsstange verbunden, auf der ein Schlagelement verschieblich geführt ist. Das Schlagelement schlägt gegen einen an der Betätigungsstange befestigten Anschlag an, so daß der auf diesen Anschlag aufgebrachte Stoß unmittelbar über den Werkzeugkopf und den Prothesenschaft auf den Prothesen-Hauptkörper übertragen wird, so daß sich dieser aus dem Knochen löst.

In der Betätigungsstange ist bevorzugt eine Stellstange verschieblich geführt, die mit dem Werkzeugkopf so verbunden ist, daß die Stellkraft auf ein äußeres Stellglied des Werkzeugkopfes aufgebracht wird. Die Stellstange ist in der hohl ausgebildeten Betätigungsstange verschieblich geführt, bevorzugt durch ein Gewinde, so daß durch Verdrehen eines mit der Stellstange verbundenen Knopfes die Stellstange axial gegen das äußere Stellglied geführt wird und dieses in axialer Richtung vorschiebt. Nach Übertragung durch die weiteren Stellglieder erreicht somit die durch die Stellstange aufgebrachte Stellkraft das Sperrglied.

In einer weiteren Ausführungsform, die auf die genannte Ausführungsform mit Kugel oder Rollen Bezug nimmt, dient die Stellstange bevorzugt zugleich zur Einstellung der Eingriffsposition des Sperrglieds zwecks Anpassung an unterschiedliche Prothesenschaftdurchmesser und zu dessen Verriegelung. Hier entfällt ein mit einem Stellglied verbundener Hebel und die zur Betätigung des Sperrglieds auf die Stellglieder aufgebrachte Stellkraft wirkt in entsprechender Dimensionierung auch zur Verspannung des Sperrglieds in die Verriegelungsposition und zur Verriegelung des Sperrglieds in dieser Position. Zu diesem Zweck kann beispielsweise die Stellstange, die in der Betätigungsstange geführt ist, so ausgebildet werden, daß sie durch einen Exzenterhebel oder dergleichen gespannt und entspannt werden kann.

In einer weiterhin bevorzugten Ausführungsform ist an einem Ende der Stellstange ein Hebelglied angeordnet, das mit der Stellstange derart in Wirkverbindung steht, daß eine Verschwenkung des Hebelgliedes die Stellstange in Richtung des Werkzeugkopfes vorschiebt. Auf diese Weise wird die durch das Hebelglied erzeugte Stellkraft längst der Stellstange und entlang der Stellglieder auf das Sperrglied übertragen. Die Anordnung eines Hebelgliedes an einem dem Werkzeugkopf gegenüberliegenden Ende der Stellstange schafft die Möglichkeit einer besonders platzsparenden und ergonomisch wirkungsvollen Betätigung des Ausschlagwerkzeuges.

Durch Verschwenkung des Hebelgliedes kann die Stellstange in Richtung des Werkzeugkopfes vorgespannt oder gelöst werden.

Bevorzugt ist das Hebelglied abnehmbar, so daß auch alternative Vorspannungseinrichtungen aufgesetzt werden können.

Weitere Vorteile, Merkmale und Anwendungsmöglichkeiten der vorliegenden Erfindung ergeben sich aus der nachfolgenden Beschreibung eines bevorzugten Ausführungsbeispiels in Verbindung mit der Zeichnung.

Fig. 1 zeigt einen Querschnitt des auf dem Prothesenschaft montierten erfindungsgemäßen Ausschlagwerkzeugs.

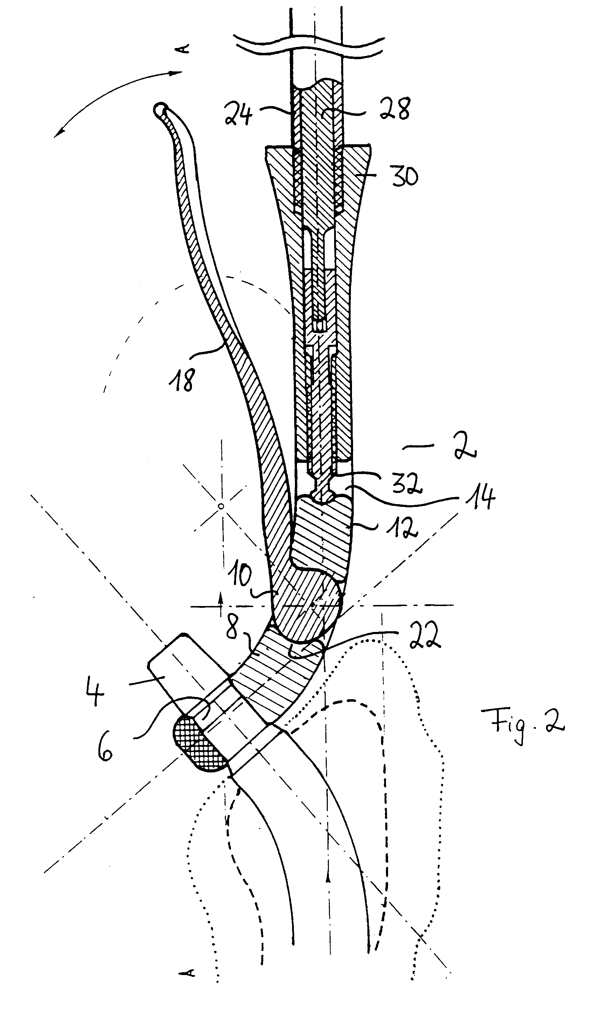

Fig. 2 zeigt eine Einzelansicht des montierten Werkzeugkopfes im Querschnitt.

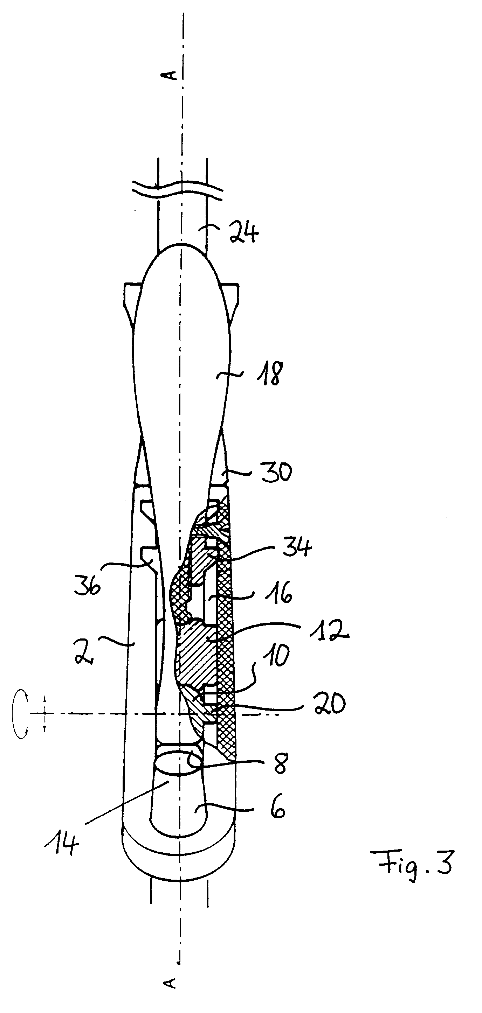

Fig. 3 zeigt den Werkzeugkopf von Fig. 2 in Aufsicht und teilweise in Aufriß.

Fig. 4 zeigt eine modifizierte Ausführungsform des Werkzeugkopfes mit Rollen.

Fig. 5 zeigt eine alternative Ausführungsform von Fig. 1 im Querschnitt.

Das in den Figuren dargestellte Ausschlagwerkzeug ist zur Befestigung an einem Prothesenschaft 4 einer Gelenkprothese bestimmt, die in einem Knochen eingesetzt bzw. eingebaut ist und durch das Ausschlagwerkzeug aus diesem entfernt werden soll.

Das Ausschlagwerkzeug besteht aus einem am Prothesenhals 4 befestigbaren Werkzeugkopf 2 und einer damit verbundenen Betätigungsstange 24, auf der ein Schlagelement 26 verschieblich geführt ist. Mit der Stange 24 ist ferner ein Anschlag 31 fest verbunden, gegen den das Schlagelement 26 zur Erzeugung eines axial gerichteten Stoßes geführt wird.

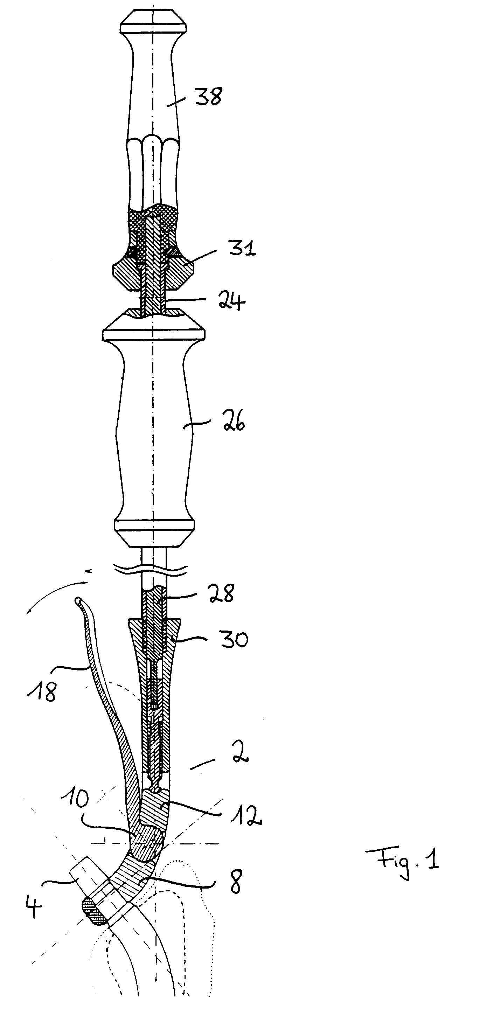

Der Werkzeugkopf 2 hat hufeisenförmige bzw. gabelförmige Gestalt mit einer Längsausnehmung 14, die zur Aufnahme verschiedener Stellglieder 8, 10, 12 bestimmt ist, und einem gegenüber der Längsausnehmung 14 verbreiterten Durchgang 6, der zur Aufnahme des Prothesenschaftes 4 bestimmt ist. Der Durchgang 6 hat kreisförmigen Querschnitt mit Ausnahme des Schnittbereichs mit der Längsausnehmung 14. In den der Längsausnehmung 14 zugewandten Seitenwänden des Werkzeugkopfes 2 sind zwei gegenüberliegende längsgestreckte elliptisch gekrümmte Nuten 16 gebildet, die zur Führung der Stellglieder 8, 10, 12 dienen. Der Werkzeugkopf 2 ist, wie in Fig. 1 zu sehen, elliptisch stetig gekrümmt bzw. gebogen, um eine zwischen dem Prothesenschaft 4 und dem daran anschließenden Prothesenhauptkörper gebildete Krümmung auszugleichen, um die durch die Betätigungsstange 24 übertragene Stoßkraft in axialer Richtung des Prothesenhauptkörpers zu übertragen. Die Nuten 16 sind der Krümmung des Werkzeugkopfes 2 angepaßt und haben ebenfalls einen gebogenen Verlauf.

In die Ausnehmung 14 wird zunächst ein Sperrglied 8 eingeführt, das gegenüberliegende Längsstege hat, die zur Aufnahme in den entsprechenden Innennuten 16 des Werkzeugkopfes 2 bestimmt sind. Dadurch läßt sich das Sperrglied 8 axial in den Werkzeugkopf 2 einführen. Da die Nuten bis zum Ende des Durchgangs 6 im Werkzeugkopf 2 geführt sind, endet die Bahn des in dem Werkzeugkopf 2 eingeführten Sperrglieds 8 am Ende der Längsausnehmung 14 bzw. im Bereich der (gedachten) Ergänzungslinie des Querschnitts des Durchgangs 6. Das Sperrglied 8 läßt sich jedoch noch weiter in den Durchgang 6 hinein vorschieben, um auch Prothesenschafte mit kleinerem Durchmesser wirksam zu verriegeln. Zur Anpassung an den kreisförmigen Querschnitt des Prothesenschaftes 4 besitzt das Sperrglied 8 eine im Querschnitt kreisbogenförmige Auflauffläche zur Anlage an dem Prothesenschaft 4.

Hinter dem Sperrglied 8 wird ebenfalls axial ein mit einem Hebel 18 versehenes Stellglied 10 eingeführt, das zwei gegenüberliegende Führungsnasen 20 aufweist, mit denen es in den Nuten 16 des Werkzeugkopfs 2 geführt ist. Der Hebel 18 dient zur Verspannung des Sperrglieds 8 von einer Freigabeposition, in der der Prothesenhals 4 in den Durchgang 6 eingeführt werden kann, in eine Verriegelungsposition, in der das Sperrglied 8 gegen den Prothesenhals 4 gespannt und in dieser Stellung verriegelt ist. Zu diesem Zweck weist das mit dem Hebel 18 verbundene Stellglied 10 eine exzentrische Auflauffläche 22 auf, die bei Verschwenkung des Hebels 18 zu einem wachsenden axialen Vorschub des Sperrglieds 8 und anschließendem Einrasten des Sperrglieds 8 in einem im wesentlichen planen Abschnitt der Auflauffläche 22 führt.

Hinter dem Stellglied 10 wird ein äußeres Stellglied 12 mit seinen zwei gegenüberliegenden Führungsstegen auf den Innennuten 16 des Werkzeugkopfs 2 aufgesteckt. Das Stellglied 12 bildet den Abschluß der beweglichen Stellglieder, die somit durch Beaufschlagung des äußeren Stellglieds 12 über Vermittlung des mittleren Stellglieds 10 das Sperrglied 8 in seiner Position voreinstellen. Die Freigabeposition des Sperrglieds 8 läßt sich somit durch axiale Druckbeaufschlagung des äußeren Stellglieds 10 verändern. Am Abschluß des Werkzeugkopfs 2 wird hinter dem äußeren Stellglied 12 ein Verbindungs- und Abschlußstück 30 auf den Werkzeugkopf aufgeschoben. Das Verbindungsstück 30 weist seitlich vorspringende Zähne auf, die zur Aufnahme in entsprechend geformten im Werkzeugkopf 2 gebildeten Ausnehmungen 36 bestimmt sind. Das Verbindungsstück 30 wird somit von oben in den Werkzeugkopf 2 eingeführt und schließt die im Werkzeugkopf 2 gebildete Längsausnehmung 14 an dem dem Durchgang 6 gegenüberliegenden Ende ab. Zur Betätigung des äußeren Stellglieds 12 ist in dem Verbindungsstück 30 eine längsgestreckte Bohrung 32 gebildet, die zum Eingriff einer Stellstange 28 bestimmt ist.

Das Verbindungsstück 30 ist axial endseitig mit der Betätigungsstange 24 verbunden, wie in Fig. 1 gezeigt. In einem Hohlkanal der Betätigungsstange 24 ist die Stellstange 28 axial geführt, die durch die Bohrung 32 des Verbindungsstücks 30 hindurchgreift, um in Anlage gegen das äußere Stellglied 12 zu gelangen. Die Stellstange 28 ist an ihrem anderen Ende mit einem Knauf 38 verbunden, der durch Drehung die Stellstange 28 aus der im Verbindungsstück 30 gebildeten Gewindebohrung 32 herausdreht und somit die axiale Eingriffsposition des Sperrglieds 8 verändert.

Nach Einführung des Prothesenschaftes 4 in den Durchgang 6 des Werkzeugkopfes 2 wird zunächst durch Drehen des Knauffes 38 die Stellstange so weit vorgeschoben, daß das Sperrglied 8 in Anlage an den Prothesenhals 4 in dem Durchgang 6 gelangt. Der Hebel 18 befindet sich dabei in seiner vom Werkzeugkopf 2 weggerichteten Freigabeposition. Nach Anlage des Sperrglieds 8 am Prothesenschaft 4 wird der Hebel 18 aus seiner Freigabestellung in seine in Fig. 1 gezeigte Verriegelungsposition, die dem Verbindungsstück 30 benachbart bzw. parallel zum Werkzeugkopf 2 ausgerichtet ist, zurückgeschwenkt. Dabei schiebt sich die exzentrische Auflauffläche 22 des Stellglieds 10 gegen das Sperrglied 8 und verspannt dieses aus seiner Freigabeposition in seine Verriegelungsposition, wo es nach Einrasten des Sperrglieds 8 an einem im wesentlichen planen Abschnitt der Auflauffläche 22 des Stellglieds 10 einrastet. Damit ist der Werkzeugkopf 2 fest an der Prothese verriegelt und die Prothese kann nun durch Schläge des Schlagelements 26 gegen die Anlage 31 aus dem Knochen entfernt werden. Zur Lösung von Werkzeugkopf 2 und Prothesenschaft 4 wird der Hebel 18 aus seiner Verriegelungsstellung in seine vom Werkzeugkopf 2 abstehende Freigabeposition verschwenkt, in der das Sperrglied in beweglicher Anlage gegen den Prothesenschaft 4 liegt. Durch die zurückweichende Auflauffläche 22 des Hebels 18 und Drehen des Knaufes 38 bekommt das Sperrglied 8 ein Spiel, so daß der Prothesenschaft 4 bequem aus dem Durchgang 6 herausgezogen werden kann.

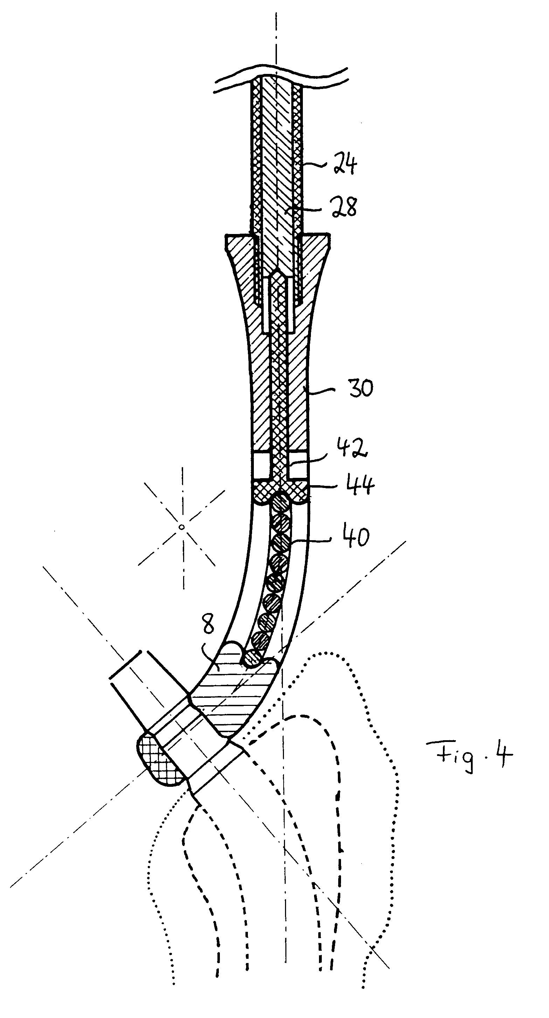

Die in Fig. 4 gezeigte Modifikation des erfindungsgemäßen Ausschlagwerkzeugs zeigt einen mit Rollen 40 bestückten Werkzeugkopf, wobei die Rollen 40 in den im Werkzeugkopf gebildeten Nuten 16 gelagert und geführt sind. Die Rollen 40 dienen somit als hintereinander angeordnete Stellglieder, die eine axial von der Stellstange 28 zu übertragende Stellkraft auf das Sperrglied 8 übertragen. Die Rollen 40 folgen der Krümmung des Werkzeugs und sind in Wirkverbindung miteinander angeordnet.

Zur Verbindung der Stellstange 28 und der Rollen 40 ist eine im Verbindungsstück 30 geführte Schubstange 42 vorgesehen, die an ihrem einen Ende mit der Stellstange 28 verbunden ist und an ihrem anderen Ende einen mit einer Vertiefung gebildeten Kopf 44 aufweist, der zur Aufnahme der Rollen dient. Der Kopf 44 sieht eine längsgestreckte Vertiefung vor, in der die Rollen angeordnet sind und gegen die die Rollen sich abstützen. Die Stellstange 28 überträgt somit durch Vermittlung der Schubstange 42 eine axiale Einstell- und Verriegelungskraft auf die Rollen 40. Da in dieser Ausführungsform ein separates Verriegelungselement in Form eines Hebels bei den Stellgliedern fehlt, wird die Verriegelungskraft ebenso wie die Einstellkraft durch die Stellstange 28 auf die Rollen 40 und damit auf das Sperrglied 8 übertragen. Somit wird ausschließlich über einen axialen Vorschub der Stellstange 28 die Einstellung der Eingriffsposition und die Verriegelung des Sperrglieds in der Verriegelungsposition bewirkt. Die Stellstange kann beispielsweise (in nicht gezeigter Weise) mit einem Exzenterhebel verbunden sein, der durch Umlegen die Stellstange 28 axial vor- oder zurückschiebt. Der Exzenterhebel kann durch Federkraft in eine Freigabeposition vorgespannt sein.

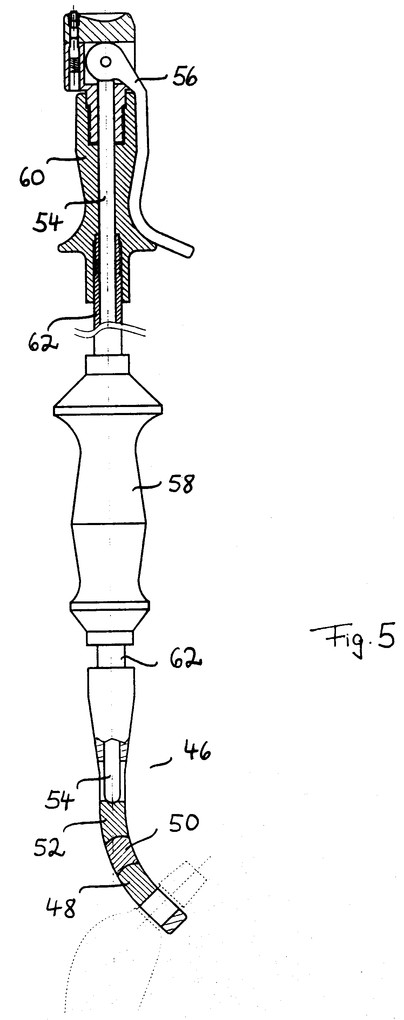

In Fig. 5 ist eine alternative Ausführungsform des Ausschlagwerkzeugs von Fig. 1 gezeigt. Ein Werkzeugkopf 46 ist mit einer Betätigungsstange 62 verbunden, auf der ein Schlagelement 58 geführt ist. Das Schlagelement 58 ist auf der Betätigungsstange 62 verschieblich geführt und kommt an einem Ende seiner Laufbahn zur Anlage gegen eine Schulter des Werkzeugkopfes 46, um auf diese Weise einen axialen Ausschlagimplus längst des Werkzeugkopfes zu übertragen. In der Betätigungsstange 62 ist in ihrem inneren Hohlraum eine Stellstange 54 beweglich geführt, die sich an ihrem einen Ende in den Werkzeugkopf 46 hinein erstreckt und zur Anlage gegen ein im Werkzeugkopf axial verschieblich geführtes Stellglied 52 gelangt. Im Werkzeugkopf 46 sind neben dem Stellglied 52 in Anlage mit diesem ein weiteres Stellglied 50 und daran anliegend ein Sperrglied 48 angeordnet, welches in einen im Werkzeugkopf gebildeten Durchgang eingreift, um einen eingeführten Prothesenschaft zu verriegeln. Die Stellkraft der Stellstange 54 führt zu einer axialen Verschiebung der Stellglieder bzw. des Sperrgliedes entlang des gekrümmten Werkzeugkopfes 46, so daß das Sperrglied seine Verriegelungsfunktion ausüben kann. An dem dem Werkzeugkopf 46 gegenüberliegenden Ende der Betätigungsstange 62 ist ein zur manuellen Handhabung geeigneter Griff 60 mit der Betätigungsstange 62 verbunden. Die Stellstange 54 streckt sich durch den Griff 60 hindurch und aus diesem heraus.

An dem Griff ist an dem der Betätigungsstange 62 gegenüberliegenden Ende ein Hebelaufsatz in eine entsprechende Vertiefung des Griffes eingesetzt, der ein Hebelglied 56 aufweist. Das Hebelglied 56 ist dem bereits vorstehend beschriebenen Hebel 18 aus Fig. 1 vergleichbar ausgebildet, so daß auf eine detallierte Beschreibung des Hebelglieds 56 verzichtet werden kann. Im Ergebnis wird durch Einrichtung einer exzentrischen Auflauffläche am Hebelglied 56 dieses in die Lage versetzt, durch Verschwenken eine axial gerichtete Stellkraft auf die Stellstange 54 auszuüben, wodurch das Sperrglied 48 im Werkzeugkopf 46 vorgeschoben und in Eingriff mit der Ausnehmung geführt wird. Der Hebelaufsatz ist abnehmbar, so daß die Stellstange 54 auch auf andere Weise vorgeschoben bzw. zurückgezogen werden kann.

Der Griff 60 ist zusammenschraubbar mit dem Führungsrohr bzw. Betätigungsstange 62 zur variablen Einstellung der Stellstange 54.

## Patentansprüche

1. Ausschlagwerkzeug für Gelenkprothesen, mit einem gekrümmten Werkzeugkopf (2), der einen Durchgang (6) zur Aufnahme eines Prothesenschaftes (4) und eine Riegeleinrichtung (8, 10, 12) aufweist, die den Prothesenschaft (4) im Durchgang (6) lösbar blockiert, wobei die Riegeleinrichtung ein in den Durchgang (6) eingreifendes Sperrglied (8) aufweist, dadurch gekennzeichnet, daß das Sperrglied (8) durch innerhalb des Werkzeugkopfes (2) axial verschieblich angeordnete Stellglieder (10, 12; 40) verstellbar ist, wobei eine auf das Sperrglied (8) wirkende Stellkraft axial entlang der Stellglieder (10, 12; 40) übertragen wird.

2. Ausschlagwerkzeug nach Anspruch 1, dadurch gekennzeichnet, daß der Werkzeugkopf (2) stetig gekrümmt ist und eine im Durchgang (6) sich öffnende axiale Längsausnehmung (14) aufweist, in der die Stellglieder (10, 12; 40) und das Sperrglied (8) angeordnet sind.

3. Ausschlagwerkzeug nach Anspruch 2, dadurch gekennzeichnet, daß die Stellglieder (10, 12) und das Sperrglied (8) durch im Werkzeugkopf (2) gebildete gegenüberliegende Nuten (16) geführt sind.

4. Ausschlagwerkzeug nach einem der vorhergehenden Ansprüche, dadurch gekennzeichnet, daß ein Stellglied (10) mit einem Hebel (18) verbunden ist, der zum Vorschub des Sperrglieds (8) in die Verriegelungsposition und zur lösbaren Blockierung des Sperrglieds in der Verriegelungsposition dient, wobei die Schwenkachse (20) des Hebels (18) im Stellglied (10) gebildet ist.

5. Ausschlagwerkzeug nach einem der vorhergehenden Ansprüche, dadurch gekennzeichnet, daß die Stellglieder durch Kugeln oder Rollen (40) gebildet sind.

6. Ausschlagwerkzeug nach einem der vorhergehenden Ansprüche, dadurch gekennzeichnet, daß mit dem Werkzeugkopf (2) eine Betätigungsstange (24, 62) verbunden ist, auf der ein Schlagelement (26, 58) verschieblich geführt ist.

7. Ausschlagwerkzeug nach Anspruch 6, dadurch gekennzeichnet, daß in der Betätigungsstange (24, 62) eine Stellstange (28, 54) verschieblich geführt ist, die mit dem Werkzeugkopf (2) so verbunden ist, daß die Stellkraft auf ein äußeres Stellglied (12) des Werkzeugskopfs (2) aufgebracht wird.

8. Ausschlagwerkzeug nach Anspruch 7, dadurch gekennzeichnet, daß die Stellstange (28, 54) zugleich zur Einstellung der Eingriffsposition des Sperrglieds (8) zwecks Anpassung an unterschiedliche Prothesenschaftdurchmesser und zur Verriegelung des Sperrglieds (8) dient.

9. Ausschlagwerkzeug nach einem der vorherhegenden Ansprüche, dadurch gekennzeichnet, daß an einem Ende der Stellstange (54) ein Hebelglied (56) angeordnet ist, das mit der Stellstange (54) derart in Wirkverbindung steht, daß eine Verschwenkung des Hebelgliedes (56) die Stellstange (54) in Richtung des Werkzeugkopfes vorschiebt.

10. Ausschlagwerkzeug nach Anspruch 9, dadurch gekennzeichnet, daß das Hebelglied (56) abnehmbar ist.

## Claims

1. A knockout tool for joint prostheses, comprising:
a curved tool head (2), the tool head having a passage (6) therethrough for receiving a prosthesis shaft; and
a locking device (8, 10, 12) to releasably block the prosthesis shaft within the passage, the locking device further comprising a blocking member (8) engaging the passage, characterized in that the blocking member (8) is adjustable by means of actuators (10, 12; 40) disposed within the tool head and axially displaceable within the tool head,
whereby an adjusting force acting upon the blocking member (8) is transmitted axially along the actuators.

2. The knockout tool of claim 1, characterized in that the tool head (2) is smoothly curved and includes an axial longitudinal recess (14) opening into the passage, in which the actuators (10, 12; 40) and the blocking member (8) are disposed.

3. The knockout tool of claim 2, characterized in that the actuators (10, 12) and the blocking member (8) are guided in opposed grooves (16) formed in the tool head (2).

4. The knockout tool according to one of the preceding claims, characterized in that one actuator (10) is connected to a lever (18) that acts to feed the blocking element (8) into a locking position and to releasably block the blocking element (8) in the locking position, wherein the inner actuator includes a pivot shaft (20).

5. The knockout tool according to one of the preceding claims, characterized in that the actuators include balls or rollers (40).

6. The knockout tool according to one of the preceding claims further comprising an actuation rod (24, 62) connected to the tool head and a hammer element (26, 58) slidably mounted on the actuation rod.

7. The knockout tool of claim 6, wherein an adjusting rod (28, 54) is slidably mounted in the actuation rod (24, 62) and is connected to the tool head (2) such that the adjusting force is brought to bear upon an outer actuator (12) of the tool head.

8. The knockout tool of claim 7, wherein the adjusting rod simultaneously acts to adjust an engagement position of the blocking element (8) for adaptation to different shaft diameters of the prosthesis shaft and to lock the blocking element (8).

9. The knockout tool according to one of the preceding claims, characterized in that a lever member (56) is disposed on an end of the adjusting rod (54) and is operatively connected to the adjusting rod such that swivelling of the lever member (56) displaces the adjusting rod toward the tool head.

10. The knockout tool of claim 9, wherein the lever member (56) is removable.

## Revendications

1. Outil d'extraction à chocs pour des prothèses de hanche, muni d'un outil incurvé (2) qui comprend un passage (6) de réception d'une tige de prothèse (4) et un dispositif de verrouillage (8, 10, 12), qui bloque de façon libérable la tige de prothèse (4) dans le passage (6), le dispositif de verrouillage comportant un organe de verrouillage (8) s'engageant dans le passage (6), caractérisé en ce que l'organe de verrouillage (8) est réglable par l'intermédiaire d'organes de réglage (10, 12; 40) disposés axialement coulissants à l'intérieur de la tête d'outil (2), de telle manière qu'une force de réglage agissant sur l'organe de réglage (8) soit transmise axialement le long des organes de réglage (10, 12; 40).

2. Outil d'extraction à chocs selon la revendication 1, caractérisé en ce que la tête d'outil (2) présente une courbure continue et comporte une cavité longitudinale axiale (14) s'ouvrant sur le passage (6) et dans laquelle sont disposés les organes de réglage (10, 12; 40) et l'organe de verrouillage (8).

3. Outil d'extraction à chocs selon la revendication 2, caractérisé en ce que les organes de réglage (10, 12) et l'organe de verrouillage (8) sont guidés par des rainures opposées (16) formées dans la tête d'outil (2).

4. Outil d'extraction à chocs selon l'une des revendications précédentes, caractérisé en ce qu'un organe de réglage (10) est relié à un levier (18) qui sert à faire avancer l'organe de réglage (8) dans la position de verrouillage et à bloquer de façon libérable l'organe de verrouillage dans la position de verrouillage, l'axe de pivotement (20) du levier (18) étant formé sur l'organe de réglage (10).

5. Outil d'extraction à chocs selon l'une des revendications précédentes, caractérisé en ce que l'organe de réglage est formé par des billes ou des rouleaux (40).

6. Outil d'extraction à chocs selon l'une des revendications précédentes, caractérisé en qu'à la tête d'outil (2) est reliée une barre d'actionnement (24, 62) sur laquelle est guidée de façon coulissante un élément de frappe (26, 58).

7. Outil d'extraction à chocs selon la revendication 6, caractérisé en ce que dans la barre d'actionnement (24, 62) est guidée de façon coulissante une barre de réglage (28, 54) qui est reliée à la tête d'outil (2), de telle façon que la force de réglage soit appliquée à un organe de réglage extérieur (12) de la tête d'outil (2).

8. Outil d'extraction à chocs selon la revendication 7, caractérisé en ce que la barre de réglage (28, 54) sert également au réglage de la position de contact de l'organe de verrouillage (8), afin de l'adapter à différents diamètres de tiges de prothèse, et au verrouillage de l'organe de verrouillage (8).

9. Outil d'extraction à chocs selon l'une des revendications précédentes, caractérisé en ce qu'à une extrémité de la barre de réglage (54), est disposé un organe de levier (56) qui est relié mécaniquement à la barre de réglage (54) de telle façon qu'un pivotement de l'organe de levier (56) provoque le coulissement de la barre de réglage (54) dans la direction de la tête d'outil.

10. Outil d'extraction à chocs selon la revendication 9, caractérisé en ce que l'organe de levier (56) est amovible.
